# EUROPEAN PATENT APPLICATION

(11) **EP 4 443 161 A1**
(43) Date of publication of application: **09.10.2024**
(21) Application number: 22901257.0
(22) Date of filing: 29.11.2022
(51) Int. Cl.: G01N 33/543

(54) **TEST KIT AND CHROMATOGRAPHY METHOD**

(30) Priority: 30.11.2021 JP 2021194134
(71) Applicant: FUJIFILM Corporation, Tokyo 106-8620 (JP)
(72) Inventor: WADA Atsuhiko, Ashigarakami-gun, Kanagawa 258-8538 (JP)
(74) Representative: Dehns Germany Partnerschaft mbB
(86) International application number: PCT/JP2022/043841
(87) International publication number: WO 2023/100825

(57) **Abstract**

An object of the present invention is to provide an assay kit for performing a chromatographic method, combined with a nasal mucus receiving medium in which a sample is easily visible and the sample is easily collected, and a chromatographic method including detecting presence or absence of a test substance in nasal mucus by using the above-descried assay kit. According to the present invention, there is provided an assay kit including (a) a nasal mucus receiving medium that includes a medium in which a visibility of a portion in contact with nasal mucus is changed in a case where nasal mucus is brought into contact with the portion, and an impermeable sheet, and (b) a chromatographic kit that includes a label substance modified with a first binding substance for a test substance, and a porous carrier having a second binding substance for the test substance or a binding substance for the first binding substance.

## Description

### Technical Field

The present invention relates to an assay kit including a nasal mucus receiving medium and a chromatographic kit. The present invention further relates to a chromatographic method including detecting presence or absence of a test substance in nasal mucus using the above-described assay kit.

### Background Art

Among immunoassay methods, an immunochromatographic method is generally utilized, because operation is easy and measurement can be performed within a short period of time. Competitive responses or sandwich-based responses are widely used as an immune response used in the immunochromatographic method. Among them, sandwich-based responses are the mainstream in the immunochromatographic method, and in a typical example thereof, the following operation is performed in order to detect a test substance composed of antigens in a specimen. First, fine particles sensitized with an antibody against an antigen which is a test substance are immobilized as solid-phase fine particles on a chromatographic carrier, or an antibody itself is directly immobilized on a chromatographic carrier, and thereby a chromatographic carrier having a reaction site is prepared. Meanwhile, labeled fine particles are sensitized with an antibody against a test substance to prepare sensitized labeled fine particles. The sensitized labeled fine particles are then chromatographically moved together with a specimen on a chromatographic carrier. By the above operation, the immobilized antibody serves as an immobilization reagent at a reaction site formed on the chromatographic carrier, and the sensitized labeled fine particles specifically bind to this immobilized antibody via an antigen which is a test substance. As a result, by visually determining the presence or absence or a degree of signals generated by the sensitized labeled fine particles trapped at the reaction site, it is possible to measure the presence or absence or an amount of a test substance in a specimen.

Patent Document 1 describes a method of identifying the presence or absence of infection with an influenza B type virus by an immunoassay, and describes that it is detected whether or not an influenza virus is present in a nasal swab. In addition, Patent Document 2 describes an assay method for respiratory infection including preparing an assay specimen suitable for a plurality of respiratory infection assays by treating a sample selected from a nasal aspirate fluid, a nasal swab fluid, and a pharyngeal swab fluid with a sample treatment liquid, assaying a part of the assay specimen using a first test tool of an immunochromatographic method for a first respiratory infection assay, and assaying a part of the assay specimen using a second test tool of an immunochromatographic method for a second respiratory infection assay. Furthermore, Patent Document 3 describes a sample sampling tool for a nasal oral excrement, which samples a nasal oral excrement as a sample, in which an inner surface side of a sampling tool main body is made of a non-transmissive material having non-transmissivity, and a wiping part consisting of an absorbent material having absorbability is provided on a part of the inner surface side of the sampling tool main body.

### Prior Art Documents

### Patent Documents

Patent Document 1: JP2010-507780A
Patent Document 2: JP2008-164403A
Patent Document 3: JP2010-276412A

### SUMMARY OF THE INVENTION

### Object to be solve by the invention

In the method of the related art in which a sample is sampled by inserting a cotton swab into the nose (nasopharyngeal swab or nasal swab), it is desirable to use a "nasal discharge sample" in which nasal mucus is sampled on a sheet or the like, since there is a concern that an infection may spread because of sneezing or the like during the sampling of the sample. However, there is a problem that it is difficult to sample a sample on a nasal discharge because a sample (nasal mucus) is difficult to be visually recognized in a "nasal discharge sample". Furthermore, in a case where nasal mucus is sampled using general pulp paper (tissue paper or the like), there is a problem that the nasal mucus is absorbed by the pulp paper, and it is difficult to sample the sample.

An object to be solved of the present invention is to provide an assay kit for performing a chromatographic method, combined with a nasal mucus receiving medium in which a sample is easily visible and the sample is easily sampled. An object to be solved of the present invention is to provide a chromatographic method including detecting presence or absence of a test substance in nasal mucus using the above-described assay kit.

### Means for solving the object

As a result of intensive studies to solve the above-described objects, the present inventors have found that the above-described obj ects can be solved by combining a nasal mucus receiving medium including a medium in which the visibility of a portion in contact with nasal mucus is changed in a case where nasal mucus is brought into contact with the portion and an impermeable sheet, with an assay kit including a chromatographic kit, and have completed the present invention.

That is, according to an aspect of the present invention, the following invention is provided.
<1> An assay kit comprising (a) a nasal mucus receiving medium that includes a medium in which a visibility of a portion in contact with nasal mucus is changed in a case where nasal mucus is brought into contact with the portion, and an impermeable sheet and (b) a chromatographic kit that includes a label substance modified with a first binding substance for a test substance, and a porous carrier having a second binding substance for the test substance or a binding substance for the first binding substance.
<2> The assay kit according to <1>, in which the visibility of the portion in contact with the nasal mucus is changed by a change in color.
<3> The assay kit according to <2>, in which the medium in which the visibility of the portion in contact with the nasal mucus is changed in a case where the nasal mucus is bought into contact with the portion contains cobalt chloride.
<4> The assay kit according to <1>, in which the visibility of the portion in contact with the nasal mucus is changed by a change in at least one of a refractive index, a reflectivity, or a transmittance.
<5> The assay kit according to <4>, in which the medium in which the visibility of the portion in contact with the nasal mucus is changed in a case where the nasal mucus is bought into contact with the medium includes a pulp paper.
<6> The assay kit according to any one of <1> to <5>, in which the chromatographic kit further includes a compound containing silver and a reducing agent capable of reducing a silver ion.
<7> The assay kit according to any one of <1> to <6>, in which the test substance is at least one of an influenza virus antigen or a coronavirus antigen.
<8> The assay kit according to any one of <1> to <7>, in which the test substance is both an influenza virus antigen and a coronavirus antigen, and both the influenza virus antigen and the coronavirus antigen are allowed to be simultaneously detected.
<9> A chromatographic method comprising detecting presence or absence of a test substance in nasal mucus using the assay kit according to any one of <1> to <8>.

### Effect of the invention

According to the chromatographic kit and the immunochromatographic method of the present invention, the sample is easily visible, and the sample can be easily sampled.

### BRIEF DESCRIPTION OF THE DRAWINGS

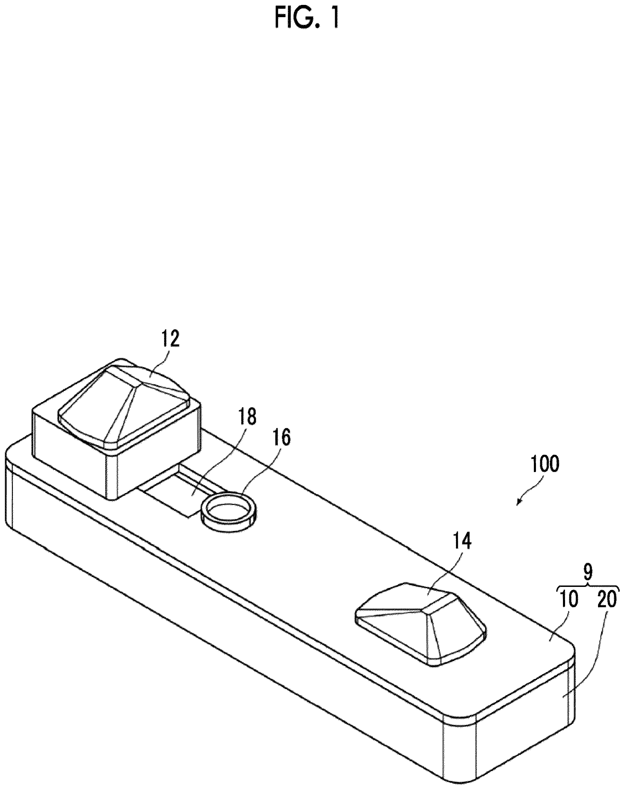

Fig. 1 is a perspective view showing an example of an immunochromatographic kit.

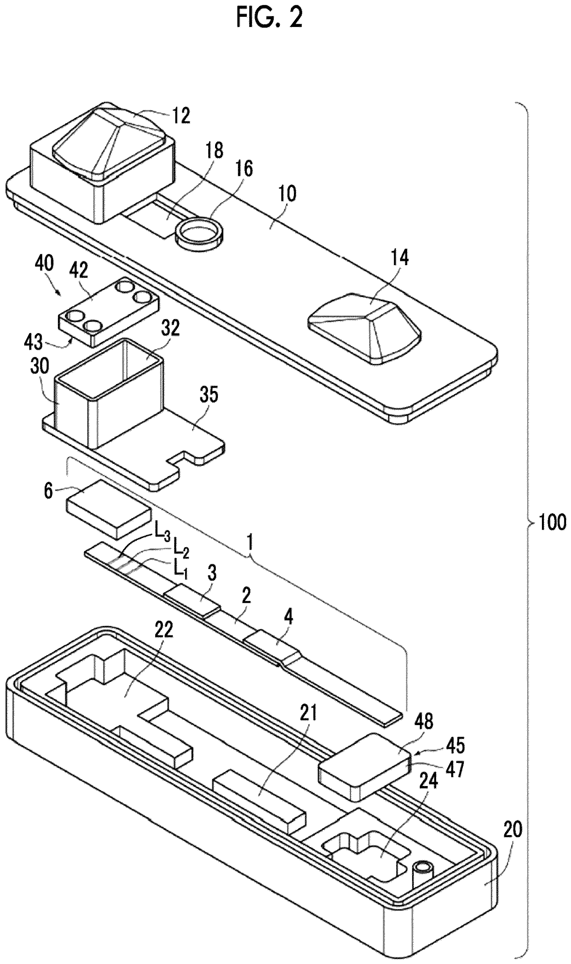

Fig. 2 is an exploded schematic perspective view showing the example of the immunochromatographic kit.

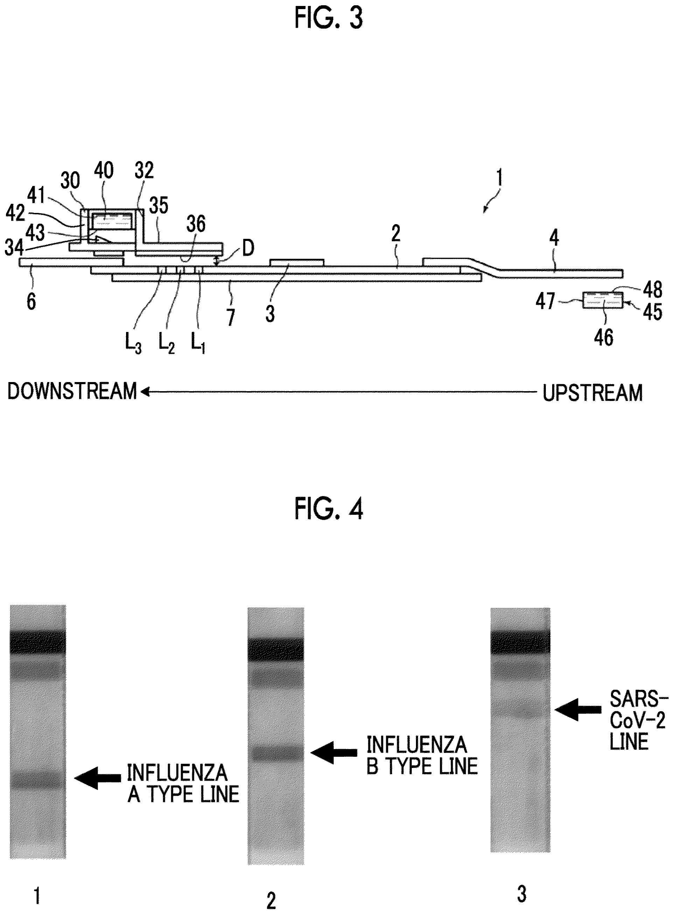

Fig. 3 is a schematic side view showing a positional relationship between an assay strip, and a first pot and a second pot.
Fig. 4 shows the measurement results of Example 1. 1 shows the result of the nasal discharge sample sampled from a healthy person and mixed with the inactivated influenza A type virus, and the result is positive for the influenza A type virus. 2 shows the result of the nasal discharge sample sampled from a healthy person and mixed with the inactivated influenza B type virus, and the result is positive for the influenza B type virus. 3 shows the result of the nasal discharge sample sampled from a healthy person and mixed with the inactivated SARS-CoV-2 virus, and the result is positive for the SARS-CoV-2 virus.

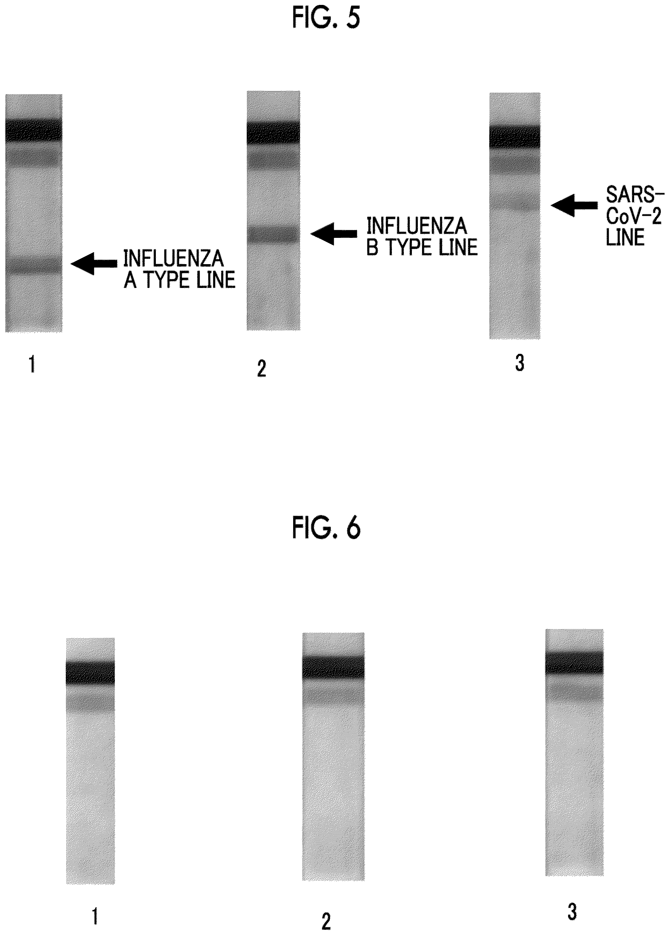

Fig. 5 shows the measurement results of Example 2. 1 shows the result of the nasal discharge sample sampled from a healthy person and mixed with the inactivated influenza A type virus, and the result is positive for the influenza A type virus. 2 shows the result of the nasal discharge sample sampled from a healthy person and mixed with the inactivated influenza B type virus, and the result is positive for the influenza B type virus. 3 shows the result of the nasal discharge sample sampled from a healthy person and mixed with the inactivated SARS-CoV-2 virus, and the result is positive for the SARS-CoV-2 virus.
Fig. 6 shows a measurement result of Comparative Example 1. 1 shows the result of the nasal discharge sample sampled from a healthy person and mixed with the inactivated influenza A type virus, 2 shows the result of the nasal discharge sample sampled from a healthy person and mixed with the inactivated influenza B type virus, and 3 shows the result of the nasal discharge sample sampled from a healthy person mixed with the inactivated SARS-CoV-2 virus, and all of 1 to 3 are negative.

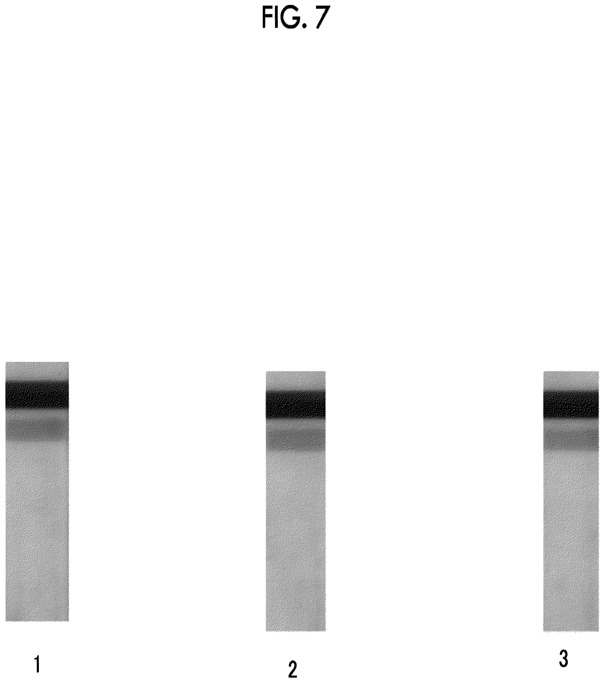

Fig. 7 shows a measurement result of Comparative Example 2. 1 shows the result of the nasal discharge sample sampled from a healthy person and mixed with the inactivated influenza A type virus, 2 shows the result of the nasal discharge sample sampled from a healthy person and mixed with the inactivated influenza B type virus, and 3 shows the result of the nasal discharge sample sampled from a healthy person mixed with the inactivated SARS-CoV-2 virus, and all of 1 to 3 are negative.

### DESCRIPTION OF THE EMBODIMENTS

Hereinafter, embodiments of the present invention will be specifically described.

In the present specification, the numerical range indicated by using "to" means a range including numerical values described before and after "to" as a minimum value and a maximum value, respectively.

The assay kit according to the embodiment of the present invention is an assay kit including (a) a nasal mucus receiving medium that includes a medium in which a visibility of a portion in contact with nasal mucus is changed in a case where nasal mucus is brought into contact with the portion, and an impermeable sheet and (b) a chromatographic kit that includes a label substance modified with a first binding substance for a test substance, and a porous carrier having a second binding substance for the test substance or a binding substance for the first binding substance.

The chromatographic method according to the embodiment of the present invention a chromatographic method including detecting presence or absence of a test substance in nasal mucus using the above-described assay kit according to the embodiment of the present invention.

In the present invention, the assay kit includes the chromatograph capable of confirming that the nasal mucus is reliably collected in the nasal mucus receiving medium, and capable of reliably sampling the collected nasal mucus and performing a sample assay in the nasal mucus. The nasal mucus receiving medium includes a member in which the visibility of a portion in contact with the nasal mucus is changed, and an impermeable sheet.

### <Member in which visibility of portion in contact with nasal mucus is changed>

The member which can be used in the present invention and in which a visibility of a portion in contact with the nasal mucus is changed is a member in which the visibility of a degree capable of visually identifying a portion in contact with the nasal mucus and a portion not in contact with the nasal mucus is changed by a change of color, a change in refractive index, a change in reflectivity, or a change in transmittance.

As the member in which the visibility is changed by the change in color, a member containing cobalt chloride is preferable. The cobalt chloride is a compound which is changed from blue to red in a case of being brought into contact with the nasal mucus by the following reaction.

CoCl₂ + 6H₂O ⇆ [Co(H₂O)₆]Cl₂

As the member containing cobalt chloride, pulp paper is preferable, and the preferred pulp paper is not particularly limited as long as it is possible to blow the nasal mucus and the nasal mucus can be collected in the presence of the impermeable sheet, but filter paper or toilet paper such as tissue paper is preferably used. These members can be used by being impregnated with cobalt chloride.

In addition, as the member in which the visibility of a degree capable of visually identifying a portion in contact with the nasal mucus and a portion not in contact with the nasal mucus is changed by the change in refractive index, the change in reflectivity, and the change in transmittance, pulp paper can be preferably used, and filter paper or toilet paper such as tissue paper can be more preferably used. Originally, there is a large difference in refractive index between air and pulp, and the nasal mucus can be recognized as white by reflecting light at various angles. However, in a case where the nasal mucus is absorbed by the pulp paper, the air layer existing in the thickness direction of the pulp paper with water having a refractive index of about 1.33 is occupied with water. Therefore, the intensity of light recognized by the viewer is reduced because of a change in difference in refractive index at the interface between the pulp, water, and air and a decrease in the total area of the interface between with the air having a refractive index of 1.0, and the portion in which the nasal mucus is present can be visually recognized as gray apparently.

### <Impermeable sheet>

The impermeable sheet that can be used in the present invention is a sheet-like material having a property of not allowing the moisture, which is a main component of the nasal mucus, to permeate, and is used for easily collecting the nasal mucus from the surface side by being bonded to a pulp paper absorbing the runny nose or a nasal mucus absorption material having a material that can cause a change of color to makes the presence of the nasal mucus visible, and making the nasal mucus locally present on the surface side impregnated with the nasal mucus. This impermeable sheet can be used by being bonded with a sheet-like material that absorbs nasal mucus. The impermeable sheet has a function of retaining a blown nasal mucus on the surface side, and thus has a function of allowing a patient to sample nasal mucus by blowing the nose in a state of being bonded to a sheet-like material that absorbs nasal mucus, and is preferably a soft material to make it easier to blow the nasal mucus. Therefore, as the material of the impermeable sheet, polyvinylidene chloride (PVDC), polyvinyl chloride (PVC), polymethylpentene (PMP), or polyethylene (PE) is preferable. In addition, the use of an impermeable sheet in which polyethylene (PE) and nylon (NYLON) are laminated is also preferable.

In order to facilitate the sampling of the nasal mucus in the medium using the impermeable sheet, the thickness of the impermeable sheet is preferably thin in a range where the permeation of water can be blocked, and preferably 1 µm or more and 100 µm or less, more preferably 1 µm or more and 50 µm or less, and still more preferably 1 µm or more and 25 µm or less.

As a method of bonding the member in which a visibility of a portion in contact with the nasal mucus is changed and that has a function of absorbing the nasal mucus, such as pulp paper, to the impermeable sheet, the member and the impermeable sheet can be bonded to each other using an adhesive. The type of the adhesive which can be used is not particularly limited as long as it is an adhesive that does not dissolve in nasal mucus after the bonding and does not interfere with the assay of the sample in the nasal mucus.

### <Chromatograph>

In general, a chromatographic method is a technique of simply, rapidly, and specifically determining and measuring a test substance by the following technique. That is, a binding substance-immobilized membrane (porous carrier) capable of having a label substance-trapping region having at least one detection site having a binding substance (specifically, an antibody) capable of binding to a test substance is used as a stationary phase. On this porous carrier, a liquid containing a label substance modified with a first binding substance against a test substance is moved chromatographically as a moving layer to reach the label substance-trapping region having the detection site while the test substance and the label substance specifically bind to each other. The technique is a technique of qualitatively and quantitatively analyzing the presence of a test substance in a test specimen visually or using an appropriate device by utilizing that, in the detection site of the label substance-trapping region, a complex of the test substance and the label substance specifically binds to an immobilized second binding substance, and thereby the label substance is concentrated in the second binding substance only in a case in which the test substance is present in a test specimen.

In a preferred aspect of the chromatographic method according to the embodiment of the present invention, using two kinds of amplification reagents used for amplifying signals of a label substance, specifically, a compound containing silver and a reducing agent capable of reducing silver ions, it is possible to amplify signals by an amplification reaction using, as a nucleus, a complex of a label substance and a test substance bound to an immobilization reagent on a label substance-trapping region, and as a result, it is possible to achieve high-sensitivity. According to the present invention, rapid chromatography with high-sensitivity can be performed.

### <Test specimen>

The test specimen that can be analyzed in the assay kit and the chromatographic method according to the embodiment of the present invention is not particularly limited as long as the specimen may contain a test substance, and is preferably nasal mucus.

The test substance according to the embodiment of the present invention is not particularly limited, and examples thereof can include at least one of an influenza virus antigen or a coronavirus antigen. The test substance may be both an influenza virus antigen and a coronavirus antigen, and both the influenza virus antigen and the coronavirus antigen may be simultaneously detected. For example, since it is also conceivable that the coronavirus infection and the influenza virus infection are simultaneously prevalent, it is important to differentiate between the coronavirus and the influenza virus from the viewpoint of treatment and isolation.

### <Pretreatment of test specimen>

In the chromatographic method according to the embodiment of the present invention, it is possible to use the test specimen as it is, or in a form in which the test specimen has been concentrated by an appropriate method, in a form in which appropriate ingredients for pretreatment are added to the test specimen, in a form of an extraction liquid obtained by extracting the test specimen using an appropriate solvent for extraction, in a form of a diluent solution obtained by diluting an extraction liquid with an appropriate diluent, or in a form of in which an extraction liquid has been concentrated by an appropriate method. As the extractant used in the present invention, it is possible to use a solvent (for example, water, a saline, a buffer solution, or the like) used in an ordinary immunological assay or a hydrophilic organic solvent capable of directly causing an antigen-antibody reaction by being diluted with such solvent.

### <Configuration>

In the chromatographic kit according to the embodiment of the present invention, a chromatographic strip can be incorporated and used. The chromatographic strip that can be used is not particularly limited as long as it is a chromatographic strip that can be used in general chromatographic methods.

The chromatographic strip that can be used in the present invention has a label substance-holding region and a label substance trapping region that are arranged in this order from the upstream direction to the downstream direction in the development direction of a test specimen. In a preferred aspect, the chromatographic strip further has a region containing a coloring reagent. A more preferred aspect of the present invention is an aspect in which the region containing a coloring reagent is located in the downstream direction of the label substance-trapping region. Furthermore, an aspect, in which a specimen addition pad, a label substance-holding pad having a label substance-holding region (for example, gold colloid antibody-holding pad), an antibody-immobilized membrane that is a porous carrier (for example, an antibody-immobilized membrane having a label substance-trapping region), and a water absorption pad are disposed in this order on a pressure-sensitive adhesive sheet, is preferably used. The antibody-immobilized membrane that is a porous carrier has a label substance-trapping region that is a region having at least one detection site on which an antibody that specifically binds to *Mycobacterium tuberculosis* is immobilized, or as desired, the antibody-immobilized membrane may further have a control site (sometimes referred to as a control region) which is a region in which an antibody for control or an antigen is immobilized.

The label substance-holding pad which has the label substance-holding region and can be used in the present invention can be prepared by preparing a suspension containing the label substance, applying the suspension to an appropriate water absorption pad (for example, glass fiber pad), and then drying the suspension.

### <Label substance>

As the label substance used in the present invention, it is preferable to use a label substance containing a metal as a label used for labeling the first binding substance against a test substance. The label substance is more preferably a metal particle. As the type of metal that can be used in the present invention, noble metals such as gold, silver, and platinum, iron, lead, copper, cadmium, bismuth, antimony, tin, or mercury can be preferably used, or compounds thereof can be used. Noble metals such as gold, silver, and platinum can be more preferably used. The label substance containing a metal that can be used in the present invention is preferably in the form of a metal colloid label or a metal sulfide label. In the present invention, as the metal colloid label, platinum colloid, gold colloid, silver colloid, iron colloid, aluminum hydroxide colloid, or the like can be preferably used, and as the metal sulfide label, each of sulfides of iron, silver, lead, copper, cadmium, bismuth, antimony, tin, or mercury can be preferably used. In the present invention, platinum colloid, gold colloid, or silver colloid can be more preferably used, and gold colloid can be most preferably used. In a case where gold colloidal particles are used as a metal colloid label, commercially available gold colloidal particles may be used. Alternatively, the gold colloidal particles can be prepared by a conventional method, for example, a method of reducing chloroauric acid with sodium citrate (Nature Physical Science, 241 (1973), 20, or the like).

The average particle diameter of the metal colloid is preferably about 1 nm to 500 nm, more preferably 3 to 100 nm, and particularly preferably 5 to 60 nm. The average particle diameter of the metal colloid used in the present invention can be measured with a commercially available particle size distribution analyzer or the like. As particle size distribution measurement methods, optical microscopy, confocal laser microscopy, electron microscopy, atomic force microscopy, a static light scattering method, laser diffractometry, a dynamic light scattering method, centrifugal sedimentation, electric pulse sensing, chromatography, an ultrasonic attenuation method, and the like are known, and devices compatible with the principles of these methods are commercially available.

As a method of measuring an average particle diameter, a dynamic light scattering method can be preferably used because of a particle size range and ease of measurement. Examples of commercially available measurement devices using dynamic light scattering include NANOTRAC UPA (Nikkiso Co., Ltd.), dynamic light scattering-type particle size distribution analyzer LB-550 (HORIBA, Ltd.), a particle size analyzer FPAR-1000 for a concentrated solution (Otsuka Electronics Co., Ltd.), and the like. In the present invention, a median diameter (d = 50) measured at a measurement temperature of 25°C is adopted.

According to the present invention, in chromatography using a metal colloid label or metal sulfide label, another metal alloy label (hereinafter sometimes referred to as a metallic label), or a metal-containing polymer particle label as a label substance for detection, signals of the metallic label can be amplified. Specifically, in a case where, after formation of a complex of a test substance and a label for detection, silver ions supplied from a compound containing silver such as an inorganic silver salt or an organic silver salt are brought into contact with a reducing agent capable of reducing silver ions, the silver ions are reduced by the reducing agent, and thereby silver particles are generated, the silver particles are deposited on a metallic label with the metallic label as a nucleus, and therefore, the metallic label is amplified, and analysis of the test substance can be performed with high-sensitivity. That is, in the chromatographic method of the embodiment of the present invention, the silver particles generated by the reducing action of silver ions by the reducing agent are used to perform a reaction for depositing on a label of an immune complex, and signals thus amplified are analyzed.

### <Binding substance>

The label substance is modified with the first binding substance for a test substance in the present invention. The first binding substance may be any binding substance as long as it is a compound, which has an affinity for a test substance, such as an antibody against a test substance (antigen), an antigen against a test substance (antibody), an aptamer against a test substance (protein, low-molecular-weight compound, and the like).

The chromatographic kit according to the embodiment of the present invention includes the second binding substance for the test substance or the binding substance for the first binding substance in label substance-trapping region. The second binding substance for a test substance may be any binding substance as long as it is a compound, which has an affinity for a test substance, such as an antibody against a test substance (antigen), an antigen against a test substance (antibody), an aptamer against a test substance (protein, low-molecular-weight compound, and the like). In addition, the second binding substance and the first binding substance may be different from each other or may be the same as each other. The binding substance for the first binding substance may be a test substance itself or may be a compound having a site recognized by the first binding substance. Examples thereof include a compound obtained by binding of a derivative of a test substance and a protein (for example, BSA or the like), and the like.

It is preferable that either or both of the first binding substance and second binding substance be an antibody.

In the chromatographic method of the embodiment of the present invention, an antibody having specificity against a test substance is not particularly limited. For example, it is possible to use antisera prepared from animal sera immunized with the test substance, immunoglobulin fractions purified from antiserum, monoclonal antibodies obtained by cell fusion using animal spleen cells immunized with the test substance, or fragments thereof [for example, F(ab')2, Fab, Fab', or Fv] or a single-chain antibody (scFv or the like). The preparation of these antibodies can be carried out by a conventional method.

In the present invention, a method of modifying the label substance using the first binding substance can be performed according to, for example, a conventionally known method described below (for example, The Journal of Histochemistry and Cytochemistry, 30, 7 (1982) 691-696) in a case of binding a metal colloid and a specific binding substance to each other. As a specific example, a metal colloid and a specific binding substance (for example, an antibody) are mixed in an appropriate buffer solution at room temperature for 5 minutes or longer. After the reaction, a precipitate obtained by centrifugation is dispersed in a solution containing a dispersant such as polyethylene glycol, and thereby a desired specific binding substance labeled with the metal colloid can be obtained.

As the binding substance, for example, an antibody for detecting an influenza A type virus, an antibody for detecting an influenza B type virus, or an antibody for detecting a SARS-CoV-2 virus can be used.

The antibody for detecting an influenza A type virus is not particularly limited as long as it can detect the influenza A type virus with high sensitivity, and a commercially available antibody can be used. Specifically, as an example, Anti-Influenza A SPTN-5 7307 manufactured by Medix Biochemica Oy is available.

The antibody for detecting an influenza B type virus is not particularly limited as long as it can detect the influenza B type virus with high sensitivity, and a commercially available antibody can be used. Specifically, as an example, MONOTOPE aby Influenza B Virus (nuclear) Purified 1131 manufactured by ViroStat, Inc. can be used.

The antibody for detecting a SARS-CoV-2 virus is not particularly limited as long as it can detect the SARS-CoV-2 virus with high sensitivity, and a commercially available antibody can be used. Specifically, as an example, an antibody manufactured by ACROBiosystems Co., Ltd. can be used.

In the present invention, a method of modifying the label substance using the first binding substance can be performed according to, for example, a conventionally known method described below (for example, The Journal of Histochemistry and Cytochemistry, 30, 7 (1982) 691-696) in a case of binding a metal colloid and an antibody to each other. As a specific example, a metal colloid and an antibody are mixed in an appropriate buffer solution at room temperature for 5 minutes or longer. After the reaction, a precipitate obtained by centrifugation is dispersed in a solution containing a dispersant such as polyethylene glycol, and thereby an antibody labeled with the metal colloid can be obtained.

### <Porous carrier>

As the porous carrier that can be used in the present invention, a nitrocellulose carrier (such as a nitrocellulose membrane), a cellulose membrane, an acetyl cellulose membrane, a polysulfone membrane, a polyether sulfone membrane, a nylon membrane, a glass fiber, a non-woven fabric, a cloth, a thread, and the like are particularly preferable.

In the present invention, a label substance-trapping region of the porous carrier has a detection site on which the second binding substance against the test substance is immobilized. The second binding substance against the test substance may be directly immobilized on a part of the porous carrier by a physical or chemical bond, and thereby the detection site is formed, or the second binding substance may physically or chemically bind to fine particles such as latex particles, these fine particles are trapped on a part of the porous carrier and immobilized, and thereby the detection site is formed. The porous carrier is preferably used after immobilizing the second binding substance against the test substance thereon and then subjecting the porous carrier to a non-specific adsorption prevention treatment such as a treatment with an inactive protein. The porous carrier of the present invention can also be preferably used in a form of having a plurality of bonding sites, and as desired, it may further have the above-mentioned control site as a part of the label substance-trapping region.

### <Label substance-holding pad>

In the present invention, an aspect in which a label substance-holding pad having a label substance-holding region, preferably a gold colloid-holding pad, is incorporated into the chromatographic kit and used is preferable. As a material of the label substance-holding pad, for example, cellulose filter paper, glass fiber, non-woven fabric, and the like can be preferably used, and it is possible to obtain the label substance-holding region by impregnating a certain amount of the label substance prepared as described above and drying it.

### <Specimen addition pad>

It is preferable to use the chromatographic kit according to the embodiment of the present invention by further incorporating a specimen addition pad thereto. For the specimen addition pad, an aspect in which the specimen-added pad has not only a function of receiving an added specimen containing a test substance but also has a function of filtering insoluble particles and the like in the specimen is preferable. Examples of the material of the specimen addition pad include materials having uniformity, such as cellulose filter paper, glass fiber, polyurethane, polyacetate, cellulose acetate, nylon, and cotton cloth. In addition, in order to prevent the test substance in the sample from being non-specifically adsorbed onto the material of the specimen addition pad during the analysis and lowering the accuracy of the analysis, it is also possible to pretreating the material configuring the specimen addition portion by a non-specific adsorption prevention treatment before use. In the present invention, the specimen addition pad may also serve as the label substance-holding pad having the label substance-holding region.

### <Water absorption pad>

In the present invention, it is preferable to use the chromatographic kit by incorporating a water absorption pad thereto. The water absorption pad is a site that physically absorbs an added specimen by chromatographic migration and also absorbs and removes unreacted label substances that are not insolubilized in a detection part of a chromatographic carrier, and water absorbent materials such as cellulose filter paper, non-woven fabric, cloth, and cellulose acetate are used. Because a chromatographic speed after a chromatographic tip end portion of the added specimen reaches the water absorption pad depends on materials, sizes, and the like of the water absorption pad, it is possible to set a speed that is suitable for measurement of the test substance by selecting materials, sizes, and the like of the water absorption pad.

### <Coloring reagent for detecting reducing agent capable of reducing silver ions>

In the chromatographic kit according to the embodiment of the present invention, a coloring reagent may be carried by a porous carrier.

In the present invention, it is preferable to use, for example, a compound that reacts with ions and forms color as the coloring reagent for detecting the reducing agent capable of reducing silver ions. Although a first amplification reagent will be described later in the present specification, a compound that reacts with Fe²⁺ ions and forms color can be used as a coloring reagent for the first amplification reagent in a case where the first amplification reagent is a reagent containing divalent iron ions (Fe²⁺), for example. As the compound that reacts with Fe²⁺ ions and forms color, it is possible to use a compound capable of forming color by forming a complex with Fe²⁺ ions. As specific examples of the compound that reacts with Fe²⁺ ions and forms color, it is possible to use compounds having a phenanthroline skeleton [for example, 1,10-phenanthroline, 5-methyl phenanthroline, 5-nitrophenanthroline, bathophenanthroline (4,7-diphenyl-1,10-phenanthroline), bathophenanthroline disulfate, and the like], or compounds having a bipyridine skeleton [for example, 2,2'-bipyridine and the like], and compounds having a phenanthroline skeleton can be preferably used. In addition, in a case in which a pH of an aqueous solution containing a test specimen and a pH of an aqueous solution containing the first amplification reagent are different from each other, it is possible to preferably use a reagent in which tint is changed because of structural change occurring due to H+ ions in order to detect the first amplification reagent. Particularly, in a case in which the aqueous solution containing the first amplification reagent is acidic (where a pH is lower than 7, and a concentration of H+ ions is high), as an pH indicator for an acidic region, it is preferable to appropriately select compounds and the like (for example, diazo-based coloring reagents such as methyl orange, methyl red, congo red, and methyl yellow, and sultone-based coloring reagents such as thymol blue, bromocresol green, bromocresol purple, and bromothymol blue), which react with H+ ions and forms color and which are well-known coloring reagents, in accordance with the pH of the aqueous solution containing the amplification reagent. Among them, 1,10-phenanthroline, bathophenanthroline, or bromocresol green can be more preferably used.

The coloring reagent is preferably a coloring reagent that does not substantially move in the porous carrier in a case where any of an aqueous solution containing a test specimen or an aqueous solution containing the reducing agent reducing silver ions is spread. Accordingly, LogP (partition coefficient in water and octanol) of the coloring reagent is preferably 4.0 or more, and more preferably 5.0 or more. An actual measurement value may be used as LogP, but a calculation value obtained from a chemical structure or the like can also be used as a simple judging method. As a method of calculating LogP, a calculation method used in ChemDraw Pro version 12 of Cambridge Soft is preferable. Responsiveness and LogP (according to ChemDraw Pro version 12) of representative coloring reagents are shown in Table 1.

**[Table 1]**

| Compound name | Responsiveness | LogP |
|---|---|---|
| 2,2'-bipyridine | Fe²⁺ responsive | 1.88 |
| Bathophenanthroline disulfate | Fe²⁺ responsive | 0.52 |
| 1,10-phenanthroline | Fe²⁺ responsive | 2.2 |
| 5-methyl phenanthroline | Fe²⁺ responsive | 2.69 |
| 5-nitrophenanthroline | Fe²⁺ responsive | 2.34 |
| Thymol blue | pH responsive | 4.01 |
| Methyl orange | pH responsive | 2.95 |
| Methyl red | pH responsive | 3.63 |
| Congo red | pH responsive | 3.63 |
| Methyl yellow | pH responsive | 4.76 |
| Bathophenanthroline | Fe²⁺ responsive | 5.55 |
| Bromocresol green | pH responsive | 7.99 |
| Bromocresol purple | pH responsive | 6.33 |
| Bromothymol blue | pH responsive | 8.8 |

A region having the coloring reagent is preferably located downstream of the label substance-trapping region having the detection site of the porous carrier. As a method of holding the coloring reagent in the chromatographic kit, there are a method of immersing a water absorption pad to be described later in a coloring reagent solution and drying under reduced pressure, a method of linearly applying in a downstream direction from a label substance-trapping region of an insoluble carrier, and the like.

In a case where the coloring reagent substantially moves in the insoluble carrier in a case where the aqueous solution containing the test specimen or the aqueous solution containing the first amplification reagent is spread, it is preferable that the coloring reagent be contained in the water absorption pad and used.

In a case where the coloring reagent does not substantially move in the insoluble carrier in a case where the aqueous solution containing the test specimen or the aqueous solution containing the first amplification reagent is spread, it is preferable to cause the insoluble carrier having the label substance-trapping region to carry the coloring reagent.

In the present invention, an aspect in which the coloring reagent is carried by the insoluble carrier is more preferable because then it is possible to display arrival of the first amplification reagent in the label substance-trapping region with a smaller time lag.

In the present invention, in a case where a region to which a test specimen containing a test substance is added, and a label substance-trapping region are provided in this order from an upstream direction to a downstream direction with respect to a development direction of the test specimen containing the test substance, an upstream direction and a downstream direction with respect to the development direction of the test specimen containing the test substance are defined in a case where the test specimen is spread by utilizing capillarity, suction power in a case where the water absorption pad is used, or the like. In a specific aspect of the present invention, in a case where a test specimen and the like are spread from a label substance-holding region toward a label substance-holding region, a direction of the label substance-holding region is defined as an upstream direction, and a direction of the label substance-trapping region is defined as a downstream direction.

In a preferred aspect of the present invention, the first amplification reagent of the two kinds of amplification reagents used for amplifying signals of a label substance trapped in the label substance-trapping region is spread from the upstream direction of the label substance-trapping region to the downstream direction of the label substance-trapping region to detect physical or chemical changes in the region having the coloring reagent, and thereby it is possible to confirm that the label substance-trapping region is filled with the first amplification reagent. As the physical or chemical changes in the region having the coloring reagent, it is possible to detect changes in color formation or fluorescence caused by a reaction between the first amplification reagent and the coloring reagent. Color formation can be preferably detected. Such physical or chemical changes may be detected visually or may be detected using a detection device.

### <Method for immunological assay>

A sandwich method which is a specific embodiment for the chromatographic method of the embodiment of the present invention will be described below.

The sandwich method is not particularly limited. In this method, for example, a test substance can be analyzed by the following procedure. First, a first binding substance for a test substance and a second binding substance for a test substance are prepared in advance. In addition, a label substance is modified in advance with the first binding substance. In a case where the second binding substance is immobilized on an appropriate chromatographic carrier (porous carrier) (for example, nitrocellulose membrane, glass fiber membrane, nylon membrane, cellulose membrane, and the like) so as to serve as a label substance-trapping region, and this region is brought into contact with a test specimen (or an extraction liquid thereof) that may contain a test substance, binding to the second binding substance (for example, an antigen-antibody reaction with the second binding substance) occurs in a case where the test substance is present in the test specimen. In a case where an excess amount of label substances modified with the first binding substance is further brought into contact with the region at the same time of binding of the test substance and the second binding substance, or after binding thereof, a complex consisting of the immobilized second binding substance and the label substances modified with the second binding substance against the test substance is formed in a case where the test substance is present in the test specimen.

In the sandwich method, it is possible to determine the presence or absence of the test substance in the test specimen or measure an amount thereof by removing a label substance that did not form an immune complex after the reaction of the immobilized second binding substance with the test substance, and the test substance with the first binding substance with which the label substances are modified is completed, and thereafter, by for example, observing a label substance-trapping region of an insoluble carrier as it is, and detecting or quantitatively determining a label substance. In the present invention, for example, a reducing agent and a silver ion-containing compound are supplied to amplify and detect the signal from the label substance forming such complex.

### <Amplification reagent>

The amplification reagent is a reagent capable of causing signal amplification by catalytically reacting by the action of the label substance or test substance and generating a colored compound or inducing luminescence or the like. The amplification reagent can be used in the form of a solution containing a reagent, that is, as an amplification liquid. Examples thereof include a silver ion solution that causes precipitation of metallic silver on a metal label by physical development, a solution of a phenylenediamine compound and a naphthol compound that turns into a colorant by the action of a peroxidase label and hydrogen peroxide, and the like.

For details, a so-called developer can be used as an amplification liquid containing an amplification reagent, where developers are described in general books in the field of photographic chemistry (for example, "Fundamentals of Photograph Engineering (Revised) - Silver Halide Photography-" (edited by the Society of Photography and Imaging of Japan, Corona Publishing Co., Ltd.), "Chemistry of Photography" (by Akira SASAI, Shashinkogyo Publishing Company), and "Latest Prescription Handbook" (by Shinichi KIKUCHI et al., Amiko Publishing Company)). It is possible to use any developer as an amplification liquid without particular limitation as long as it is a so-called physical developer which contains silver ions in the liquid and in which the silver ions in the liquid are reduced mainly by metal colloids and the like which form the core of development.

The chromatographic kit according to the embodiment of the present invention may preferably further include a compound containing silver and a reducing agent capable of reducing silver ions. That is, specifically, for example, as the amplification liquid, it is possible to use the first amplification liquid a reducing agent capable of reducing silver ions and the second amplification liquid containing a silver-containing compound in combination.

In the present invention, as for the two amplification reagents used for amplifying signals of the label substance trapped in the label substance-trapping region, it is preferable that a first amplification reagent be preliminarily incorporated into a first amplification liquid and a second amplification reagent be preliminarily incorporated into a second amplification liquid, and that the first amplification liquid and the second amplification liquid be sequentially added to perform amplification. The first amplification liquid is preferably added to a pad which is for liquid sending of a reducing agent solution and is located in an upstream direction of a label substance-holding pad and a specimen addition pad.

Hereinafter, the reducing agent capable of reducing silver ions contained in the first amplification liquid, and the compound containing silver contained in the second amplification liquid will be described.

### <Compound containing silver>

As the silver-containing compound, it is possible to use a silver ion-containing compound, for example, an organic silver salt, an inorganic silver salt, or a silver complex. The silver-containing compound is preferably a silver ion-containing compound having high solubility in a solvent such as water, and examples thereof include silver nitrate, silver acetate, silver lactate, silver butyrate, silver thiosulfate, and the like. Among these, silver nitrate is particularly preferable. The silver complex is preferably a silver complex coordinated to a ligand having a water-soluble group such as a hydroxyl group or a sulfone group, and examples thereof include hydroxythioether silver and the like.

Generally, the content of silver contained in the inorganic silver salt or the silver complex is 0.001 mol/m² to 0.2 mol/m², and preferably 0.01 mol/m² to 0.05 mol/m².

### <Reducing agent capable of reducing silver ion>

As the reducing agent capable of reducing silver ions, any inorganic or organic material or a mixture thereof can be used as long as these can reduce silver ions into silver.

Preferred examples of the inorganic reducing agent include a reducing metal salt and a reducing metal complex salt that can undergo valence change by metal ions such as Fe²⁺, V²⁺, and Ti³⁺. In a case where an inorganic reducing agent is used, oxidized ions need to be removed or made harmless by the formation of a complex or reduction. For example, in a system in which Fe²⁺ is used as a reducing agent, by using citric acid or EDTA, it is possible to make Fe³⁺ harmless by forming a complex of Fe³⁺ which is an oxide. In this system, an inorganic reducing agent is preferably used, and a metal salt of Fe²⁺ is more preferably used.

It is also possible to use a main developing agent used in a light-sensitive silver halide photographic material of a wet-type (such as methyl gallate, hydroquinone, substituted hydroquinone, 3-pyrazolidones, p-aminophenols, p-phenylenediamines, hindered phenols, amidoximes, azines, catechols, pyrogallols, ascorbic acid (or derivatives thereof), and leuco colorants), and other materials obvious to those who are skilled in the technology in the present field, such as a material disclosed in US6020117A.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly, D-, L-, or D,L-ascorbic acid (and an alkali metal salt thereof) or isoascorbic acid (or an alkali metal salt thereof) is preferable, and a sodium salt is a preferred salt. A mixture of these reducing agents can be used as necessary.

### <Other auxiliaries>

As other auxiliaries of the amplification liquid, buffers, preservatives such as antioxidants or organic stabilizers, and rate regulators may be included. As the buffer, it is possible to use, for example, acetic acid, citric acid, sodium hydroxide, or salts of any of these substances, or a buffer formed of tris(hydroxymethyl)aminomethane or other buffers used in general chemical experiments. These buffers are appropriately used to adjust the pH of the amplification liquid to an optimum pH thereof. In addition, as an antifogging agent, an alkylamine can be used as an additive, which is particularly preferably dodecylamine. Furthermore, in order to improve the solubility of these additives, a surfactant can be used, which is particularly preferably C₉H₁₉-C₆H₄-O-(CH₂CH₂O)₅₀H.

As a method of spotting the amplification reagent on the chromatographic kit, a method is preferable in which a reducing agent solution as the first amplification liquid is spotted on a pad for liquid sending of the reducing agent solution, a silver ion solution as the second amplification liquid is spotted from above on a region including a label substance-trapping region, and the silver ion solution is infiltrated in a thickness direction of an insoluble carrier.

As a method of incorporating the two kinds of amplification reagents into the chromatographic kit, there is a method of disposing a pot containing a solution containing each of the amplification reagents above a site at which each of the amplification reagents is spotted. It is preferable that the reducing agent solution (first amplification liquid) be placed on the pad for liquid sending of the reducing agent solution, and the pot containing the silver ion solution (second amplification liquid) be placed immediately above a hole filled with the silver ion solution. By disposing in this manner, the liquid flows by pushing each pot and can be spotted on a predetermined site.

### <Immunochromatographic kit>

In the immunochromatographic kit according to the embodiment of the present invention, the label substance modified with the first binding substance for the test substance may be provided on the porous carrier in advance, or alternatively, the label substance modified with the first binding substance for the test substance may be provided separately from the porous carrier. In this case, the label substance modified with the first binding substance for the test substance, which is provided separately from the porous carrier, can be measured by a method such as a method of mixing the label substance with a test specimen and then spreading the mixture on the porous carrier.

The immunochromatographic kit according to the embodiment of the present invention may further include a compound containing silver and a reducing agent capable of reducing silver ions.

The immunochromatographic kit according to the embodiment of the present invention may include a housing case including, therein, the porous carrier having a reaction site, the compound containing silver, and the reducing agent capable of reducing silver ions.

The immunochromatographic kit according to the embodiment of the present invention may further include pots each having a breakable member, in which the compound containing silver and the reducing agent capable of reducing silver ions may be respectively sealed in the pots. In this case, the pots can be broken by an external force.

Fig. 1 is a schematic perspective view illustrating an immunochromatographic kit 100, and Fig. 2 is an exploded schematic perspective view of the immunochromatographic kit 100 of Fig. 1.

As illustrated in Fig. 1 and Fig. 2, in the immunochromatographic kit 100 of the present embodiment, a housing case 9 includes an assay strip 1 that has an insoluble carrier 2 having an assay region of a test substance and is for spreading a specimen solution, and a first pot 40 and a second pot 45 which are for amplifying a detection signal in the assay region, which respectively include a surface having a sheet member, and in which a first amplification liquid 41 and a second amplification liquid 46 are sealed, respectively. The housing case 9 is formed by including a lower case 20 including an accommodation portion 21 in which the assay strip 1 is disposed, an upper case 10 joined to the lower case 20 at a peripheral edge, and an intermediate member 30 disposed between the upper case 10 and the lower case 20. It is noted that in the description of this immunochromatographic kit 100, the upper case 10 side is defined as the upper side, and the lower case 20 side is defined as the lower side.

The intermediate member 30 has a pot accommodation portion 32 which accommodates the first pot 40 and which has, on a bottom surface, an amplification liquid-filled hole for dropwise addition of the first amplification liquid 41 onto the insoluble carrier 2. In addition, a protrusion-shaped breaking portion 34 that breaks a sheet member 43 is provided at a position in the first pot 40, facing the sheet member 43 in the pot accommodation portion 32. In the present example, the first pot 40 is disposed above the pot accommodation portion 32 so that the surface having the sheet member 43 is the lower surface, and the breaking portion 34 is provided on the bottom surface of the pot accommodation portion 32 facing the sheet member 43 (refer to Fig. 3).

In addition, a flow channel forming portion 35 extending toward the downstream side of the bottom surface of the pot accommodation portion 32 of the intermediate member 30 is provided. The flow channel forming portion 35 is disposed to coincide with an upper position of an assay region L₁, a confirmation region L₂, and an amplification label region L₃, and it is formed of a transparent material in order to make these regions L₁ to L₃ visible.

The upper case 10 includes a first convex deformation portion 12 that is deformed toward the first pot 40 side by applying a pressing force to the portion facing the first pot 40 from the outside and breaks the sheet member 43 of the first pot 40 by the breaking portion 34 of the intermediate member 30. In addition, the upper case 10 includes a second convex deformation portion 14 that is deformed toward the second pot 45 side by applying a pressing force to the portion facing a second pot 45 from the outside and breaks the sheet member 48 of the second pot 45.

In addition, the upper case 10 includes an opening pore 16 for dropwise addition of a specimen solution, and the specimen solution is dropwise added from the opening pore 16 onto a label holding pad 3 of the assay strip 1. In a case of adjusting the position of the label holding pad 3 so that the positions of the opening pore 16 and the label holding pad 3 match with each other, a specimen solution can be reliably spotted on the label holding pad 3. In addition, the upper case 10 includes an observation window 18 for visually recognizing the three regions L₁ to L₃, at a position corresponding to the flow channel forming portion 35 of the intermediate member 30.

In the lower case 20, as an accommodation portion in which the assay strip 1 is disposed, a porous carrier accommodation portion 21 on which the porous carrier 2 is mounted is provided, and an absorption pad accommodation portion 22 on which an absorption pad 6 is mounted is provided on the downstream side of the porous carrier accommodation portion. In addition, a second pot accommodation portion 24 in which the second pot 45 is accommodated is provided on the upstream side of the porous carrier accommodation portion 21.

Fig. 3 is a schematic cross-sectional view illustrating a positional relationship between the assay strip 1, the intermediate member 30, and the two pots 40 and 45. As illustrated in Fig. 3, the assay strip 1 includes the porous carrier 2 spreading the specimen solution, the label holding pad 3 having a label substance modified with a binding substance immobilized on the insoluble carrier 2, a liquid feeding pad 4 which is disposed in contact with one end of the porous carrier 2 and sends the second amplification liquid 46 to the porous carrier 2, and the absorption pad 6 disposed in contact with the other end of the porous carrier 2. The porous carrier 2 is immobilized to and supported by a back pressure-sensitive adhesive sheet 7. In addition, between the label holding pad 3 and the absorption pad 6, the porous carrier 2 has the assay region L₁, the confirmation region L₂, and the amplification label region L₃ in this order from the label holding pad 3 side.

In the present specification, there are cases in which the porous carrier 2, which has the assay region L₁, the confirmation region L₂, and the amplification label region L₃ formed thereon, is referred to as a chromatographic carrier. In addition, in the present specification, as illustrated in Fig. 3, the liquid feeding pad 4 side is defined as an upstream side and the absorption pad 6 side is defined as a downstream side.

The intermediate member 30 is positioned at an upper part on the downstream end side of the assay strip 1, and the first pot 40 is disposed in the pot accommodation portion 32 of the intermediate member 30 with the sheet member 43 facing down. The second pot 45 is accommodated below the upstream end of the assay strip 1 of the lower case 20 with the sheet member 48 facing up.

As shown in Fig. 3, a gap (clearance) D is formed between a back surface 36 of the flow channel forming portion 35 of the intermediate member 30, and the insoluble carrier 2 of the assay strip 1. The gap D is preferably in a range of 0.01 mm to 1 mm. In a case where it is 0.01 mm or more, the amplification liquid or the like can be sufficiently infiltrated, and in a case where it is 1 mm or less, the capillary force is exhibited, whereby the gap between the insoluble carrier 2 and the intermediate member 30 is uniformly filled with the first amplification liquid 41.

In the first pot 40 in which the first amplification liquid 41 is enclosed, a container 42 having an opening on one surface composed of, for example, a resin material is filled with the first amplification liquid 41, and the opening of the container 42 is covered and enclosed by the breakable sheet member 43.

Similarly, in the second pot 45 in which the second amplification liquid 46 is enclosed, a container 47 having an opening on one surface composed of, for example, a resin material is filled with the second amplification liquid 46, and the opening of the container 47 is covered and enclosed by the breakable sheet member 48.

As the breakable sheet members 43 and 48 in the first pot 40 and the second pot 45, a laminated film such as an aluminum foil or an aluminum laminate sheet is suitably used. Here, "break" refers to a state where a member is not regenerated after being ruptured.

Hereinafter, the present invention will be described in more detail with reference to examples of the present invention. The materials, amounts of use, proportions, treatment contents, treatment procedures, and the like shown in the following Examples can be appropriately modified without departing from the spirit and scope of the present invention. Accordingly, the scope of the present invention is not limited to the following specific examples.

### Examples

### (1) Production of immunochromatographic kit

### (1-1) Production of anti-influenza A type antibody-modified gold colloid as label substance modified with first substance capable of binding to test substance

1 mL of a 50 mmol/L KH₂PO₄ buffer (pH 7.5) was added to 9 mL of a gold colloid solution having a diameter of 50 nm (product number: EM. GC50, manufactured by BBI Solutions Inc.) to adjust the pH, and then 1 mL of 160 µg/mL of an anti-influenza A type monoclonal antibody (Anti-Influenza A SPTN-5 7307, manufactured by Medix Biochemica Oy) solution was added to the gold colloid solution, and stirring was carried out. After allowing to stand for 10 minutes, 550 µL of a 1% polyethylene glycol ((PEG), weight-average molecular weight (Mw.): 20,000, product number: 168-11285, FUJIFILM Wako Pure Chemical Corporation) aqueous solution was added thereto, stirring was carried out, and subsequently 1.1 mL of an aqueous solution of 10% bovine serum albumin ((BSA), Fraction V, product number: A-7906, manufactured by Sigma-Aldrich Co., LLC) was added thereto, and stirring was carried out. The solution was centrifuged (himac CF16RX, Hitachi, Ltd.) at a centrifugal acceleration of 8000 × g and 4°C for 30 minutes, then the supernatant was removed to leave about 1 mL, and the gold colloid was redispersed by an ultrasonic cleaner. Thereafter, the gold colloid was dispersed in 20 mL of a gold colloid storage solution (20 mmol/L Tris-hydrochloride (HCl) buffer (pH 8.2), 0.05% PEG (Mw: 20,000), 150 mmol/L NaCl, and 1% BSA), and centrifuged again at 8,000 × g and 4°C for 30 minutes, then the supernatant was removed to leave about 1 mL, and the gold colloid was redispersed by an ultrasonic cleaner to obtain an antibody-modified gold colloid (50 nm) solution.

### (1-2) Production of anti-influenza B type antibody-modified gold colloid as label substance modified with first substance capable of binding to test substance

1 mL of a 50 mmol/L KH₂PO₄ buffer (pH 7.5) was added to 9 mL of a gold colloid solution having a diameter of 50 nm (product number: EM. GC50, manufactured by BBI Solutions Inc.) to adjust the pH, and then 1 mL of 160 µg/mL of an anti-influenza B type monoclonal antibody (MONOTOPE aby Influenza B Virus (nuclear) Purified 1131, ViroStat, Inc.) solution was added to the gold colloid solution, and stirring was carried out. After allowing to stand for 10 minutes, 550 µL of a 1% polyethylene glycol ((PEG), weight-average molecular weight (Mw.): 20,000, product number: 168-11285, FUJIFILM Wako Pure Chemical Corporation) aqueous solution was added thereto, stirring was carried out, and subsequently 1.1 mL of an aqueous solution of 10% bovine serum albumin ((BSA), Fraction V, product number: A-7906, manufactured by Sigma-Aldrich Co., LLC) was added thereto, and stirring was carried out. The solution was centrifuged (himac CF16RX, Hitachi, Ltd.) at a centrifugal acceleration of 8000 × g and 4°C for 30 minutes, then the supernatant was removed to leave about 1 mL, and the gold colloid was redispersed by an ultrasonic cleaner. Thereafter, the gold colloid was dispersed in 20 mL of a gold colloid storage solution (20 mmol/L Tris-hydrochloride (HCl) buffer (pH 8.2), 0.05% PEG (Mw: 20,000), 150 mmol/L NaCl, and 1% BSA), and centrifuged again at 8,000 × g and 4°C for 30 minutes, then the supernatant was removed to leave about 1 mL, and the gold colloid was redispersed by an ultrasonic cleaner to obtain an antibody-modified gold colloid (50 nm) solution.

### (1-3) Production of anti-SARS-CoV-2 type antibody-modified gold colloid as label substance modified with first substance capable of binding to test substance

1 mL of a 50 mmol/L KH₂PO₄ buffer (pH 7.5) was added to 9 mL of a gold colloid solution having a diameter of 50 nm (product number: EM. GC50, manufactured by BBI Solutions Inc.) to adjust the pH, and then 1 mL of 160 µg/mL of an anti-SARS-Cov-2 monoclonal antibody (ACROBiosystems) solution was added to the gold colloid solution, and stirring was carried out. After allowing to stand for 10 minutes, 550 µL of a 1% polyethylene glycol ((PEG), weight-average molecular weight (Mw.): 20,000, product number: 168-11285, FUJIFILM Wako Pure Chemical Corporation) aqueous solution was added thereto, stirring was carried out, and subsequently 1.1 mL of an aqueous solution of 10% bovine serum albumin ((BSA), Fraction V, product number: A-7906, manufactured by Sigma-Aldrich Co., LLC) was added thereto, and stirring was carried out. The solution was centrifuged (himac CF 16RX, Hitachi, Ltd.) at a centrifugal acceleration of 8000 × g and 4°C for 30 minutes, then the supernatant was removed to leave about 1 mL, and the gold colloid was redispersed by an ultrasonic cleaner. Thereafter, the gold colloid was dispersed in 20 mL of a gold colloid storage solution (20 mmol/L Tris-hydrochloride (HCl) buffer (pH 8.2), 0.05% PEG (Mw: 20,000), 150 mmol/L NaCl, and 1% BSA), and centrifuged again at 8,000 × g and 4°C for 30 minutes, then the supernatant was removed to leave about 1 mL, and the gold colloid was redispersed by an ultrasonic cleaner to obtain an antibody-modified gold colloid (50 nm) solution.

### (1-2) Production of each antibody-modified gold colloid-holding pad as label holding pad

Each antibody-modified gold colloid produced in (1-1), (1-2), and (1-3) was diluted with water such that the Tris-HCl buffer (pH 8.2) concentration was 20 mmol/L, the PEG (Mw. 20,000) concentration was 0.05% by mass, the sucrose concentration was 5% by mass, and the optical density of the gold colloid at 520 nm was 0.1 in a case where optical path length was set to 10 mm, thereby obtaining a gold colloid coating liquid. Each glass fiber pad (Glass Fiber Conjugate Pad, manufactured by MilliporeSigma) cut in 5 mm × 300 mm was uniformly coated with 1 mL of this coating liquid and dried under reduced pressure for 24 hours, thereby obtaining an antibody-modified gold colloid-holding pad.

### (1-4) Production of chromatographic carrier

Using nitrocellulose membrane cut into 54 mm × 300 mm (with a plastic backing, HiFlow Plus HF135 (capillary flow rate = 135 sec/cm), manufactured by MilliporeSigma) as a porous carrier, an assay region (three regions), a confirmation region, and an amplification label region were formed on this membrane by a method described below, and thereby a chromatographic carrier was produced.

The long side of the membrane of 300 mm was placed downward, and an anti-influenza A type monoclonal antibody (Anti-Influenza A SPTN-5 7307, manufactured by Medix Biochemica Oy) solution prepared to have a concentration of 1.5 mg/mL was applied in a line shape at a position of 21.1 mm from the downstream side, an anti-influenza B type monoclonal antibody (MONOTOPE aby Influenza B Virus (nuclear) Purified 1131, ViroStat, Inc.) solution prepared to have a concentration of 1.5 mg/mL was applied in a line shape at a position of 19.1 mm from the downstream side, and an anti-SARS-CoV-2 monoclonal antibody (ACROBiosystems) solution prepared to have a concentration of 1.5 mg/mL was applied in a line shape at a position of 17.1 mm from the downstream side. Furthermore, an anti-mouse IgG antibody (Anti-mouse IgG (H + L), rabbit F(ab')2, product number: 566-70621, FUJIFILM Wako Pure Chemical Corporation) solution prepared to have a concentration of 0.5 mg/mL was applied in a line shape at a position 15.1 mm from the downstream side. Furthermore, bromocresol green (FUJIFILM Wako Pure Chemical Corporation) prepared to have a concentration of 30 mmol/L was applied in a line shape at a position of 13.6 mm from the downstream side. The applied membrane was dried at 50°C for 30 minutes using a hot air dryer. The membrane was immersed in a vat containing 500 mL of a blocking slution (50 mmol/L borate buffer (pH 8.5) containing 0.5% by mass casein (derived from milk, product number: 030-01505, FUJIFII,M Wako Pure Chemical Corporation), and allowed to stand for 30 minutes as it was. Then, the membrane was transferred to and immersed in 500 mL of a washing and stabilizing solution (a 50 mmol/L Tris-HCl (pH 7.5) buffer containing 0.5% by mass sucrose and 0.05% by mass sodium cholate) contained in another vat, and allowed to stand for 30 minutes as it was. The membrane was taken out from the liquid and dried at room temperature overnight.

### (1-5) Production of assay strip

The chromatographic carrier produced in (1-4) was attached to a back pressure-sensitive adhesive sheet (57 mm × 300 mm (manufactured by Adhesives Research)). Then, a double-sided tape (Nitto Denko Corporation.) having a width of 3 mm was fixed to a position 17 mm distant from the downstream short side between the short sides of the chromatographic carrier. Then, the antibody-modified gold colloid-holding pad was fixed to the chromatographic carrier such that the downstream end of the double-sided tape and the downstream end of the gold colloid-holding pad produced in (1-2) overlapped each other. A liquid feeding pad (a glass fiber pad cut in 25 mm × 300 mm (Glass Fiber Conjugate Pad, manufactured by MilliporeSigma)) was attached to the upstream side of the chromatographic carrier such that the liquid feeding pad and the chromatographic carrier overlapped with each other by 5 mm. With a guillotine cutter (CM 4000, manufactured by NIPPN TechnoCluster Inc.), the member prepared as above was cut along a direction parallel to a direction perpendicular to the 300 mm long side such that the cut piece had a width of 3 mm. In this way, 80 assay strips (without an absorption pad) were prepared.

### (1-6) Production of amplification liquid

### (1-6-1) Production of amplification liquid (reducing agent solution) to be sealed in second pot

23.6 mL of an aqueous solution of 1 mol/L iron nitrate, which was produced by dissolving iron (III) nitrate nonahydrate (FUJIFILM Wako Pure Chemical Corporation, 095-00995) in water, and 13.1 g of citric acid (FUJIFILM Wako Pure Chemical Corporation, 038-06925) were dissolved in 290 g of water. After all of the substances were dissolved, 36 mL of a nitric acid (10% by weight) solution was added thereto while stirring with a stirrer, and 60.8 g of ammonium iron (II) sulfate hexahydrate (FUJIFILM Wako Pure Chemical Corporation, 091-00855) was added thereto. The solution prepared in this way was used as a reducing agent solution which is a second amplification liquid to be sealed in a second pot.

### (1-6-2) Production of amplification liquid (silver ion solution) sealed in first pot

A silver nitrate solution (8 mL, containing 10 g of silver nitrate) and 24 mL of a 1 mol/L aqueous iron nitrate solution were added to 66 g of water. Furthermore, this solution was mixed with a solution obtained by dissolving 5.9 mL of nitric acid (10 wt%), 0.1 g of dodecylamine (FUJIFILM Wako Pure Chemical Corporation, 123-00246), and 0.1 g of a surfactant C₁₂H₂₅-C₆H₄-O-(CH₂CH₂O)₅OH in 47.6 g of water in advance. This mixed solution was used for a silver ion solution which was the first amplification liquid sealed in the first pot.

### (1-7) Production of absorption pad

Eighty glass fiber pads (glass filter paper, manufactured by ADVANTEC CO., LTD.) cut IN 12 mm × 10 mm were prepared and used as absorption pads.

### (1-8) Production of components of immunochromatographic kit

The lower case 20, the upper case 10, the intermediate member 30, the first pot 40, and the second pot 45 constituting the immunochromatographic kit 100 shown in Figs. 1 to 3 were respectively produced by injection molding using polypropylene as a material. The upper case was prepared by injection molding by using polypropylene containing 50% by mass of TAFCELENE (registered trademark), which is an olefin-based elastomer manufactured by Sumitomo Chemical Co., Ltd., as a material. It is noted that the upper case 10 includes two deformable sites (the first convex deformation portion and the second convex deformation portion), and these two deformable portions do not have a portion separated from the upper case 10, which were produced by injection molding as part of the upper case 10 in the entire boundary part.

In examples, the upper case was configured such that a first convex deformation portion 12 shown in Figs. 1 and 2 have two protrusions and a second convex deformation portion 14 has one protrusion.

### (1-9) Production of immunochromatographic kit of examples

The lower case 20, the assay strip 1 produced in (1-5), and the absorption pad 6 produced in (1-7) were fixed as illustrated in Figs. 1 to 3. Next, the first pot 40 and the second pot 45 were respectively filled with the amplification liquid 41 to be sealed in the first pot 40 produced in (1-6-2) and the amplification liquid 46 to be sealed in the second pot 45 produced in (1-6-1). The pot 45 was sealed with aluminum foil as the sheet member 48, and the pot 40 was sealed with aluminum foil as the sheet member 43. As shown in Figs. 1 to 3, the second pot 45 was attached to the lower case 20 with the sheet member 48 facing upward, and the first pot 40 was attached to the intermediate member 30 with the sheet member 43 facing downward. Thereafter, in a state where the upper case 10 and the lower case 20 were fitted such that the outer periphery thereof came into contact with each other, the contact portions between the upper case and the lower case were joined by ultrasonic welding. At this time, the welded sites were checked to make sure that all the sites are uniformly welded in a sealed state. An immunochromatographic kit was prepared in this manner.

### (1-10) Production of extraction liquid container

A diluent solution containing 0.10% by weight of casein (030-01505, FUJIFELM Wako Pure Chemical Corporation) and 0.10% by weight of Tween 80 was produced. 400 µL of the produced solution was dispensed to each polypropylene container, and the container was heat-sealed with an aluminum sheet.

### (1-11) Preparation of paper changing color with nasal mucus

(A) A paper was prepared by bonding a cobalt chloride paper to an impermeable sheet (polyethylene). The colorant of cobalt chloride changes from blue to red by being wet with water.
(B) A paper was prepared by bonding a pulp paper to an impermeable sheet (polyethylene). The reflection of the pulp paper changes and the visibility changes by being wet with water.

### (Example 1)

As a simulated sample, a nasal discharge sample sampled from a healthy person and mixed with an inactivated influenza A type virus, an inactivated influenza B type virus, or an inactivated SARS-CoV-2 virus was attached to A) cobalt chloride paper. Then, the portion to which the sample adhered changed to red, and the nasal discharge sample was easily identifiable. Furthermore, the absorption of the nasal discharge sample was suppressed by the impermeable sheet, and the sample could be easily sampled with a cotton swab. In a case where the sample is extracted by stirring the cotton swab that has been used to sample the nasal discharge sample in the extraction liquid prepared in (1-10) and the evaluation is performed using the kit prepared in (1-9), the results were obtained as positive for influenza A type in the nasal discharge sample containing the inactivated influenza A type virus, as positive for influenza B type in the nasal discharge sample containing the inactivated influenza B type virus, and as positive for SARS-CoV-2 in the nasal discharge sample containing the inactivated SARS-CoV-2 virus (Fig. 4).

### (Example 2)

As a simulated sample, a nasal discharge sample sampled from a healthy person and mixed with an inactivated influenza A type virus, an inactivated influenza B type virus, or an inactivated SARS-CoV-2 virus was attached to a sheet in which a pulp paper was bonded to a B) impermeable sheet. Then, the portion to which the sample adhered changed to gray, and the nasal discharge sample was easily identifiable. Furthermore, the absorption of the nasal discharge sample was suppressed by the impermeable sheet, and the sample could be easily sampled with a cotton swab. In a case where the impermeable sheet is not provided, the sample infiltrates the pulp paper, and the sample overflows on the opposite side to which the sample is attached because of gravity, and thus it is difficult to sample the sample. On the other hand, in a case where the impermeable sheet is bonded, the sample floats on the surface to which the sample is attached, and thus the sample can be sampled.

In a case where the sample is extracted by stirring the cotton swab that has been used to sample the nasal discharge sample in the extraction liquid prepared in (1-10) and the evaluation is performed using the kit prepared in (1-9), the results were obtained as positive for influenza A type in the nasal discharge sample containing the inactivated influenza A type virus, as positive for influenza B type in the nasal discharge sample containing the inactivated influenza B type virus, and as positive for SARS-CoV-2 in the nasal discharge sample containing the inactivated SARS-CoV-2 virus (Fig. 5).

### (Comparative Example 1)

As a simulated sample, a nasal discharge sample sampled from a healthy person and mixed with an inactivated influenza A type virus, an inactivated influenza B type virus, or an inactivated SARS-CoV-2 virus was attached to polyethylene sheet. Thereafter, a portion where the sample would have been adhered was rubbed with a cotton swab, the sample was extracted by rinsing in the extraction liquid prepared in (1-10), and the evaluation was carried out using the kit prepared in (1-9). As a result, in the nasal discharge sample containing each of the inactivated influenza A type virus, the inactivated influenza B type virus, and the inactivated SARS-CoV-2 virus, all of the results were negative (Fig. 6). It was considered that this was because a portion to which the nasal discharge sample was adhered was not clear, and thus it was not possible to sample the sample by rubbing a portion to which the sample was not adhered with a cotton swab.

### (Comparative Example 2)

As a simulated sample, a nasal discharge sample sampled from a healthy person and mixed with an inactivated influenza A type virus, an inactivated influenza B type virus, or an inactivated SARS-CoV-2 virus was attached to a pulp paper (not bonded to an impermeable sheet). Then, the portion to which the sample had adhered changed to a gray. Thereafter, a portion where color was changed to a gray was rubbed with a cotton swab, the sample was extracted by rinsing in the extraction liquid prepared in (1-10), and the evaluation was carried out using the kit prepared in (1-9). As a result, in the nasal discharge sample containing each of the inactivated influenza A type virus, the inactivated influenza B type virus, and the inactivated SARS-CoV-2 virus, all of the results were negative (Fig. 7). It was considered that the nasal discharge sample was absorbed by the pulp paper and thus the sample could not be sampled with the cotton swab.

### Explanation of References

1 assay strip
2: porous carrier
3: label holding pad (glass fiber pad)
4: liquid feeding pad
6: absorption pad
7: back pressure-sensitive adhesive sheet
9: housing case
10: upper case
12: first convex deformation portion
14: second convex deformation portion
16: opening pore for dropwise addition of specimen solution
18: observation window
20: lower case
21: porous carrier accommodation portion
22: absorption pad accommodation portion
24: pot accommodation portion
30: intermediate member
32: pot accommodation portion
34: breaking portion
35: flow channel forming portion
36: back surface of flow channel forming portion 35
40: first pot for first amplification liquid
41: first amplification liquid
42: container
43: sheet member
45: second pot for second amplification liquid
46: second amplification liquid
47: container
48: sheet member
100: immunochromatographic kit
L₁: assay region
L₂: confirmation region
L₃: amplification label region
D: gap (clearance)

## Claims

1. An assay kit comprising:
(a) a nasal mucus receiving medium that includes a medium in which a visibility of a portion in contact with nasal mucus is changed in a case where nasal mucus is brought into contact with the portion, and an impermeable sheet; and
(b) a chromatographic kit that includes a label substance modified with a first binding substance for a test substance, and a porous carrier having a second binding substance for the test substance or a binding substance for the first binding substance.

2. The assay kit according to claim 1,
wherein the visibility of the portion in contact with the nasal mucus is changed by a change in color.

3. The assay kit according to claim 2,
wherein the medium in which the visibility of the portion in contact with the nasal mucus is changed in a case where the nasal mucus is bought into contact with the portion contains cobalt chloride.

4. The assay kit according to claim 1,
wherein the visibility of the portion in contact with the nasal mucus is changed by a change in at least one of a refractive index, a reflectivity, or a transmittance.

5. The assay kit according to claim 4,
wherein the medium in which the visibility of the portion in contact with the nasal mucus is changed in a case where the nasal mucus is bought into contact with the medium includes a pulp paper.

6. The assay kit according to claim 1 or 2,
wherein the chromatographic kit further includes a compound containing silver and a reducing agent capable of reducing a silver ion.

7. The assay kit according to claim 1 or 2,
wherein the test substance is at least one of an influenza virus antigen or a coronavirus antigen.

8. The assay kit according to claim 1 or 2,
wherein the test substance is both an influenza virus antigen and a coronavirus antigen, and both the influenza virus antigen and the coronavirus antigen are allowed to be simultaneously detected.

9. A chromatographic method comprising:
detecting presence or absence of a test substance in nasal mucus using the assay kit according to claim 1 or 2.
